# EUROPEAN PATENT APPLICATION

(11) **EP 4 755 330 A1**
(43) Date of publication of application: **10.06.2026**
(21) Application number: 25220998.6
(22) Date of filing: 05.12.2025
(51) Int. Cl.: A61B 18/14, A61B 5/06, A61B 5/367

(54) **MINIMIZING THE ELECTRICAL FIELD NEAR AN ELECTRODE**

(30) Priority: 06.12.2024 US 202463728806 P; 01.12.2025 US 202519404372
(71) Applicant: Biosense Webster (Israel) Ltd., 2066717 Yokneam (IL)
(72) Inventor: BAR-TAL, Meir, 2066717 Yokneam (IL); BERGER, Abraham, 2066717 Yokneam (IL); HAZAN, Ori Emanuel, 2066717 Yokneam (IL)
(74) Representative: Carpmaels & Ransford LLP

(57) **Abstract**

A catheter assembly is configured for preventing the occurrence of at least one hotspot during operation of the catheter assembly. The catheter assembly includes a shaft and an end effector. The shaft is designed to be guided into a lumen through a delivery sheath. The electrode is printed on a substrate and is configured to deliver energy to an ablation site. The bottom surface of the electrode is coupled to the substrate and the arcuate element(s) are coupled to the upper surface of the electrode. The arcuate element(s) have a curved upper surface and extend over respective location(s). The arcuate element(s) reduce the intensity of the electrical field at their respective locations to below the arcing level when the electrode is operated in the blood, thereby preventing the occurrence of hotspots during operation.

## Description

### TECHNOLOGICAL FIELD

The presently disclosed subject matter generally relates to catheter assemblies for tissue ablation, and, more specifically, to catheter assemblies designed to prevent arcing during tissue ablation.

### BACKGROUND

Ablation techniques are widely used in various medical procedures, including the treatment of tumors, cardiac arrhythmias, and other conditions where selective tissue destruction is desired.

One technique for tissue ablation is pulsed field ablation (PFA). PFA utilizes high-voltage electric pulses to create pores in the cell membrane, a process known as electroporation, leading to cell death without significant heat generation. This mechanism provides for precise targeting of tissues, reduced collateral damage of surrounding tissue, and the ability to treat larger and more complex tissue structures than other techniques.

### OVERVIEW

PFA is typically performed using a catheter assembly which includes an end effector that is guided through a shaft to the ablation site. The end effector includes one or more electrodes printed on a substrate, which deliver energy to the ablation site. The structure and design of the end effector is of critical importance to ensuring that the tissue ablation is performed with minimal damage to surrounding tissue.

One important aspect of the electrode design addressed herein is to prevent arcing around the electrode during the ablation procedure. The high voltage and electric field typical of PFA may result in the formation of steam bubbles in the blood. If the electrical field is strong enough, an arcing phenomenon occurs, which is undesirable as it is intense and may degrade the materials constituting the electrode and cause damage to surrounding tissue.

As denoted herein, according to some aspects of the invention, the term "hotspot" means a location at which a surrounding electrical field with intensity above the arcing level occurs when the electrode is operated in blood.

Some aspects of the disclosure prevent the occurrence of hotspot(s) by coupling one or more arcuate elements to the electrode at location(s) where a hotspot is expected to develop if the electrode were not protected by the arcuate element. Such locations are denoted herein "potential hotspot locations".

Potential hotspot locations are typically characterized by corners between materials with significantly different electrical conductivity. For example, the electrical conductivity of common metal electrodes is very high (σ = 6x10⁷ S/m Siemens/meter, Siemens is 1/Ω). Current in a high conductivity electrode flows to the electrode rim with virtually no resistance, resulting in potential hotspot(s) near one or more electrode edges (e.g., at the corner of the upper surface of the electrode, the connection between the electrode edge and the substrate, etc.).

According to some aspects of the presently disclosed subject matter, one or more arcuate elements are coupled to the electrode at respective potential hotspot locations where a hotspot might occur if the electrode were in direct contact with blood. The arcuate elements reduce the intensity of the electric field at their respective locations, thereby preventing arcing.

It is desirable that the electrode remains flexible even when protected by the arcuate elements, so that the shaft may be contained within and inserted through the sheath. This may be attained by using relatively thin arcuate elements. In some examples, the arcuate elements are printed on the electrode with flexible ink. The stretch ratio λ=l\L (where l is the final length and L is the initial length) of the flexible conductive ink may be about 1,000 times the stretch ratio of a typical metal. Thus a thin flexible conductive ink may have an elongation of 10% with almost the same performance as compared to metals with only a 0.01% elongation.

Similarly, the substrate should be flexible enough to permit it to pass through the sheath with the arcuate elements present.

As described in more detail below, the materials used to form the electrode and arcuate element(s) may have similar or differing respective conductivities. The size and shape of the arcuate element(s) are adapted to the differences between these conductivities.

### BRIEF DESCRIPTION OF THE DRAWINGS

In order to better understand the subject matter that is disclosed herein and to exemplify how it may be carried out in practice, examples will now be described, by way of non-limiting example only, with reference to the accompanying drawings, in which:
FIG. 1 illustrates a catheter-based electrophysiology mapping and ablation system, according to examples of the presently disclosed subject matter;
FIG. 2 illustrates an electrode without arcuate elements coupled to a substrate;
FIGS. 3A-3B show simulation results for a flat electrode without arcuate elements coupled to a substrate;
FIGS. 4A-4B are simplified diagrams of a catheter assembly, according to respective examples of the presently disclosed subject matter;
FIG. 5 is a simplified illustration of an end effector having a balloon-shaped substrate, according to examples of the presently disclosed subject matter;
FIGS. 6A-6B are simplified cutaway views of an end effector according to examples of the presently disclosed subject matter;
FIG. 7A is a simplified flowchart of a method of manufacturing a catheter assembly having hotspot protection, according to examples of the presently disclosed subject matter;
FIG. 7B is a simplified flowchart of a method of preparing an end effector, according to examples of the presently disclosed subject matter;
FIGS. 8A-8D are simplified illustrations of an end effector, according to some examples of the presently disclosed subject matter;
FIGS. 9A-9D are simplified illustrations demonstrating a process for manufacturing an end effector, according to examples of the presently disclosed subject matter;
FIGS. 10A-10C are simplified illustrations of an end effector, according to some examples of the presently disclosed subject matter;
FIGS. 11A-11D are simplified illustrations demonstrating a process for manufacturing an end effector, according to examples of the presently disclosed subject matter;
FIG. 12 is a simplified illustration of an edge effector, according to some examples of the presently disclosed subject matter;
FIGS. 13A-13C are simplified cutaway views illustrating a lamination process for manufacturing embedded electrodes, according to an exemplary embodiment of the presently disclosed subject matter; and
FIG. 14 is a simplified cutaway view of an embedded electrode, according to an exemplary embodiment of the presently disclosed subject matter.

### DETAILED DESCRIPTION

According to aspects of the disclosure, one or more arcuate elements are coupled to the top surface (also denoted the "upper surface") of an electrode of a catheter assembly. The arcuate element(s) reduce the intensity of the electrical field surrounding their respective locations during operation in blood, relative to the intensity of the electrical field that would arise for an electrode of similar size, shape, and material but without the arcuate elements.

In the following detailed description, numerous specific details are set forth in order to provide a thorough understanding. However, it will be understood by those skilled in the art that the presently disclosed subject matter may be practiced without these specific details. In other instances, well-known methods and features have not been described in detail so as not to obscure the presently disclosed subject matter.

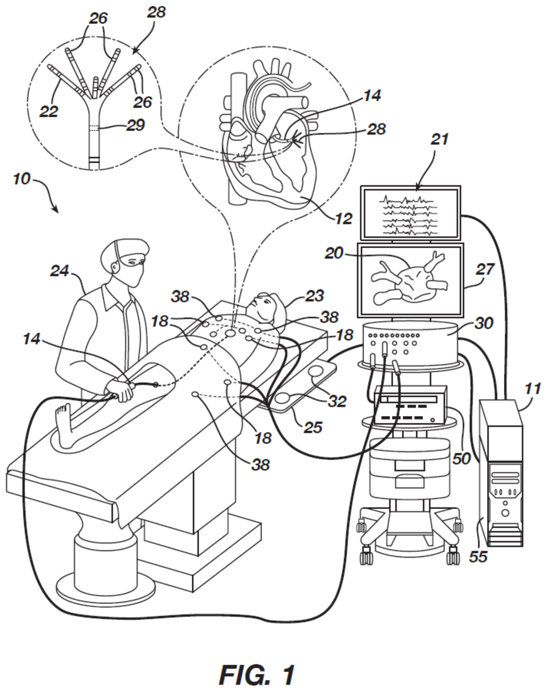

Reference is made to FIG. 1 showing an example catheter-based electrophysiology mapping and ablation system 10. System 10 includes multiple catheters, which are percutaneously inserted by a physician 24 through the patient's vascular system into a chamber or vascular structure of a heart 12. Typically, a delivery sheath catheter is inserted into the left or right atrium near a desired location in heart 12. Thereafter, one or more catheters may be inserted into the delivery sheath catheter so as to arrive at the desired location in heart 12. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 may place a distal tip 28 of catheter 14 in contact with the heart wall for sensing a target site in heart 12. For ablation, physician 24 may similarly place a distal end of an ablation catheter in contact with a target site for ablating tissue.

Catheter 14 is an exemplary catheter that includes one and preferably multiple electrodes 26 optionally distributed over a plurality of splines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location reference for location pad 25 as well as impedance-based tracking of electrodes 26. For impedance-based tracking, electrical current is directed to electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182.

A recorder 11 records and displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, other electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software stored therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

While the above-described system is directed to a catheter assembly for ablation of heart tissue, this example is non-limiting. Other aspects of the catheter assembly may be suitable for ablation of tissue in other body organs, for example for the ablation of kidney or lung tissue.

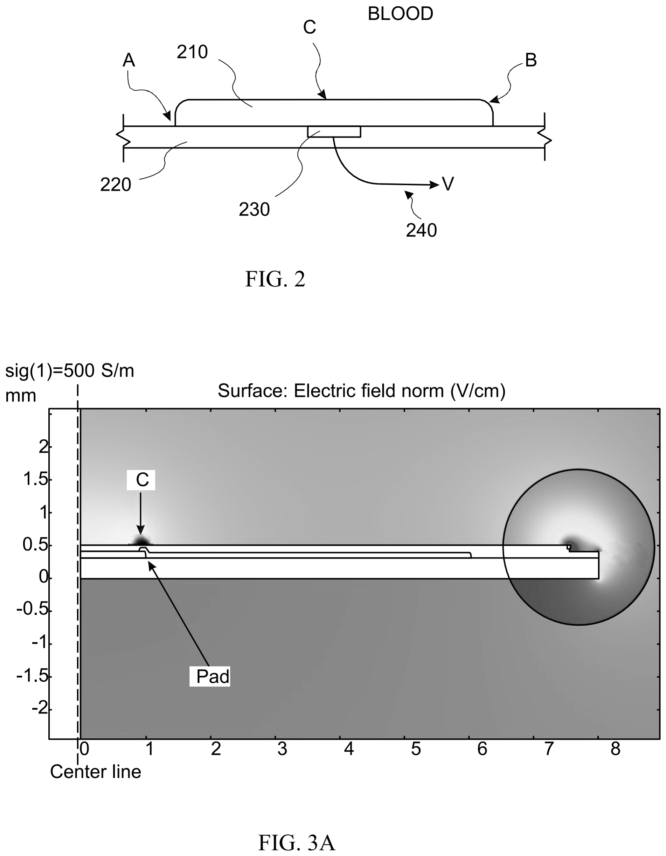

Reference is now made to FIG. 2, which is a simplified block diagram of an electrode without arcuate elements coupled to a substrate. Electrode 210 is printed on an insulating substrate 220 (such as a balloon wall). Metallic pad 230 is connected to electric wire 240, which connects to a power source (not shown) that supplies power to electrode 210.

There are several locations on electrode 210 which might develop hotspots if the electrode is operated directly in the blood. Typical hotspot locations are marked:
a) Location A: Surrounding the junction between insulating substrate 220 (e.g., balloon wall), electrode 210 and the blood.
b) Location B: Surrounding the corner of electrode 210 in the blood.
c) Location C: Above the junction of the corner of pad 230 (typically copper), electrode 210 and insulating substrate 220.

FIGS. 3A-3B show simulation results for a flat electrode with a typical material thickness of about 20µm. A high electric field is visible in all three potential hotspot locations, A, B and C.

### I. Catheter Assembly

According to some aspects of the disclosed subject matter, a catheter assembly is configured for preventing the occurrence of at least one hotspot during operation of the catheter assembly. This is achieved by coupling one or more arcuate elements to the electrode, at potential hotspot locations which might be surrounded by high intensity electric fields during the ablation procedure if the electrode were not coupled to the arcuate element(s).

For clarity, some aspects of the disclosure describe a non-limiting example of a catheter assembly having a single electrode. Other aspects according to the presently disclosed subject matter may include a catheter assembly with multiple electrodes, at least one of which is coupled to arcuate element(s) to prevent the occurrence of a strong electrical field around the electrode during operation of the catheter assembly.

The catheter assembly includes a shaft and an end effector coupled to the distal portion of the shaft. The shaft is configured to be guided into a lumen through a delivery sheath.

The end effector includes an electrode printed on a substrate and at least one arcuate element.

The electrode is configured to deliver energy to an ablation site. The bottom surface of the electrode is coupled to the substrate. The arcuate elements are coupled to the top surface of the substrate.

According to some aspects of the disclosed subject matter, the arcuate element(s) are printed on the electrode.

The arcuate element(s) extend over respective locations and reduce the intensity of the electrical field at the respective locations to below the arcing level when the electrode is operated in the blood. In this way the occurrence of hotspot(s) around the electrode during operation in blood is prevented.

In some examples, the end effector includes an arcuate element that extends over an edge of the top surface of the electrode.

In an alternate or additional aspect, the end effector includes an arcuate element that extends over an exposed connection between the bottom surface of the electrode and the substrate.

In another alternate or additional aspect, the end effector includes a conductive pad configured to supply power to the electrode, and the end effector includes an arcuate element that extends over the location of the conductive pad.

In some aspects of the disclosure, a single arcuate element extends over multiple potential hotspot locations.

For effective operation, it is desired that the surface area of the electrode remains as large as possible. In some aspects of the disclosure, the total area covered by the arcuate element(s) is less than 10% of the top surface of the electrode. In other aspects of the disclosure, the total area covered by the arcuate element(s) is less than 13% of the top surface of the electrode. In yet other aspects of the disclosure, the total area covered by the arcuate element(s) is less than 15% of the top surface of the electrode.

According to one aspect of the disclosure, the substrate is expandable (e.g., balloon-shaped). The expandable substrate may be folded so that it may be guided through the delivery sheath until the end effector reaches the ablation site. Once the ablation site is reached the substrate is inflated.

In in alternate example, the substrate is non-expandable. In a further example the substrate has a cylindrical shape.

### I.1. Conductivities

One factor that affects the capability of the arcuate element to reduce the electrical field is the respective conductivities of the electrode material and the arcuate element material.

The following terms are used herein to describe the conductivity level of a material (e.g., electrode material, arcuate element material, etc.):
1. Conductive material (metal) - electrical conductivity of 6x10⁷S/m;
2. Low conductivity material - electrical conductivity of 10⁴ to 10⁶ S/m;
3. Very low conductivity material- electrical conductivity of 100 to 5,000 S/m;
4. Extremely low conductivity material- electrical conductivity of 0.5 to 5 S/m; and
5. Nonconductive material- electrical conductivity of 10⁻¹³ S/m.

In one aspect of the disclosure, the electrode and the arcuate element are composed of the same material, thus having the same conductivity. In one example, the electrode and the arcuate element have the same conductivity, which is between 10³-10⁶ S/m. In a further aspect, the thickness of the arcuate element is within 1.8-5.2 times the thickness of the electrode.

In another aspect of the disclosure, the electrode and arcuate element are composed of different materials and the conductivity of the arcuate element is less than the conductivity of the electrode. In one example, the conductivity of the electrode is between 10³-10⁶ S/m and the conductivity of the arcuate element is within 0.5-5 S/m. In a further aspect, the thickness of the arcuate element is within 0.8-3.2 times the thickness of the electrode.

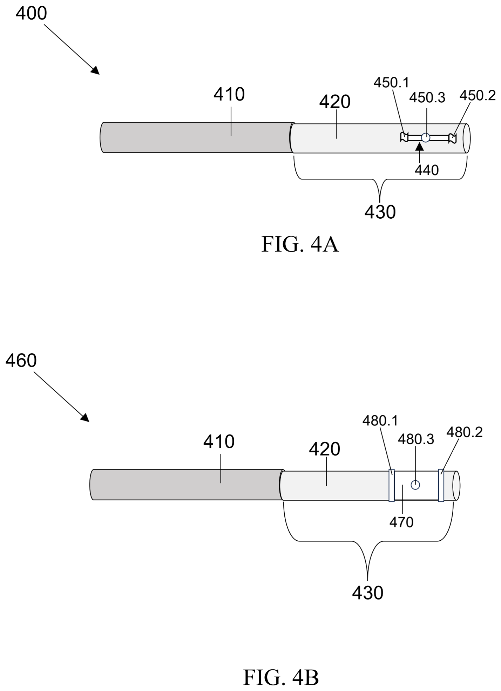

Reference is now made to FIG. 4A, which is a simplified diagram of a catheter assembly, according to examples of the presently disclosed subject matter. Catheter assembly 400 includes a shaft 410 and an end effector 430. Shaft 410 is configured to be guided into a lumen through a delivery sheath. End effector 430 is coupled to the proximal end of shaft 410 and includes electrode 440 and arcuate elements 450.1-450.3 coupled to electrode 440.

Electrode 440 is coupled to substrate 420 and is configured to deliver energy to an ablation site. Electrode 440 is shaped as a strip along a portion of the circumference of end effector 430.

The arcuate elements have curved upper surfaces and extend over respective potential hotspot locations. Arcuate elements 450.1-450.2 are located at respective edges of electrode 440. Arcuate element 450.3 is located above a conductive pad (not shown) delivering voltage to electrode 440.

The arcuate element(s) prevent the occurrence of hotspots around electrode 440 during the ablation procedure by reducing the intensity of the electrical field at their respective locations to below the arcing level.

Reference is now made to FIG. 4B, which is a simplified diagram of a catheter assembly, according to examples of the presently disclosed subject matter. Catheter assembly 460 includes a shaft 410 and an end effector 430. Shaft 410 is configured to be guided into a lumen through a delivery sheath. End effector 430 is coupled to the proximal end of shaft 410 and includes electrode 470 and arcuate elements 480.1-480.3 coupled to electrode 470. Electrode 470 is coupled to substrate 420 and is configured to deliver energy to an ablation site.

Electrode 470 and arcuate elements 480.1-480.2 surround the circumference of end effector 430. The arcuate elements have curved upper surfaces and extend over respective potential hotspot locations. Arcuate elements 480.1-480.2 are located at respective edges of electrode 470. Arcuate element 480.3 is located above a conductive pad (not shown) delivering voltage to electrode 470.

The arcuate element(s) prevent the occurrence of hotspots around electrode 470 during the ablation procedure by reducing the intensity of the electrical field at their respective locations to below the arcing level.

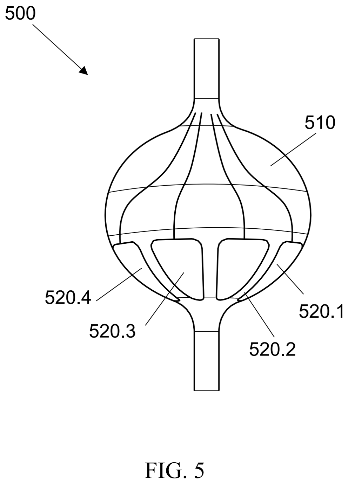

Reference is now made to FIG. 5, which is a simplified illustration of an end effector 500 having a balloon-shaped substrate 510, according to examples of the presently disclosed subject matter. Electrodes 520.1-520.4 are printed on balloon-shaped substrate 510. For heart ablation procedures, the balloon may be about 30 mm in diameter, with a relatively thick catheter that needs to be navigated to the heart with a sheath. The balloon is flexible enough to be folded and passed through the sheath. It is therefore desirable for the printed electrodes 520.1-520.4 and their coupled arcuate elements (not shown) to be as thin as possible and made of flexible ink as each layer of printing may stiffen the balloon.

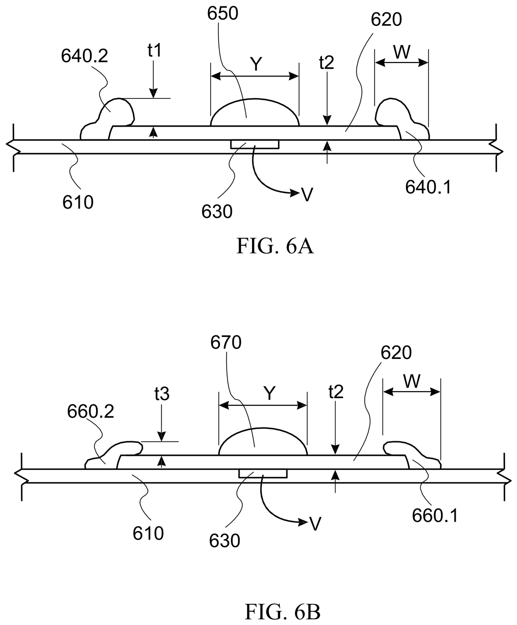

Reference is now made to FIGS. 6A-6B, which are simplified cutaway views of an end effector according to examples of the presently disclosed subject matter. In FIG. 6A the electrode and arcuate elements are made of the same material. In FIG. 6B the electrode and arcuate elements are made of different materials having different respective conductivities.

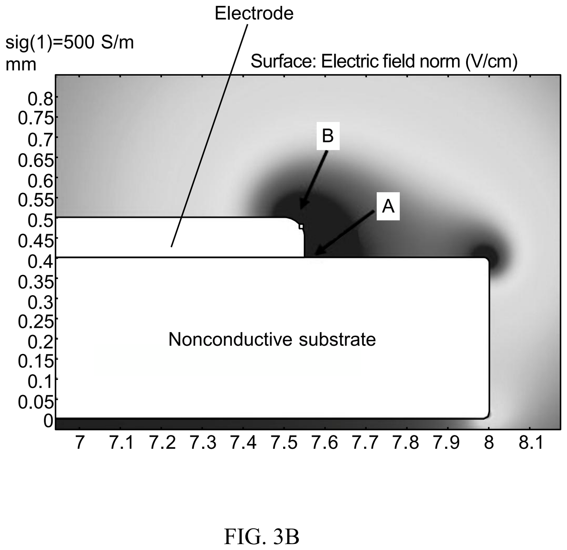

Referring to FIG. 6A, electrode 620 is printed from a material with low to very low conductivity, with a layer thickness t2 of about 10 microns (range of 5-20 microns). Arcuate elements 640.1, 640.2 and 650 are formed of the same material as electrode 620. Arcuate element 640.1 covers a slight portion of the top surface of electrode 620, the entire height of electrode 620, and slightly covering the top surface of substrate 610 (e.g., balloon), ensuring potential hotspot locations (e.g., locations A and B in Fig. 3B) are fully covered. This arcuate cover creates a larger radius at the corner, reducing the electric field by at least half. In one example, the width W of the arcuate element is about 0.5-1.0 mm (range 0.3-2.0 mm), applied approximately equally on both sides/ends of the top surface of electrode 620 (or around the rim of a closed shape). The height t1 of the arcuate element above the surface of electrode 620 is about 50 microns (range 20 to 80 microns), making the total thickness above the substrate surface about 60 microns.

A second, similarly shaped and sized arcuate element 640.2 is coupled to the opposite edge of electrode 620.

A third arcuate element 650 is coupled above pad 630 which supplies the high voltage. Arcuate element 650 is dome-shaped with a thickness t1 and a diameter Y slightly larger than the pad, (e.g., if the pad diameter is 2 mm, the arcuate element diameter Y may be 3 mm).

Referring to FIG. 6B, electrode 620 is printed from a material with low to very low conductivity with a layer thickness t2, of about 10 microns. The electrode edges are covered with an arcuate elements 660.1- 660.2 made from a material with extremely low conductivity (i.e. electrical conductivity of 0.5 to 5 S/m). The arcuate element thickness t3 is about 20 microns and an average width W of about 0.5-1.0 mm. Arcuate element 670 above pad 630 is also made from the extremely low conductivity material and has a thickness t3 of about 20 microns.

### II. Method of Manufacturing a Catheter Assembly

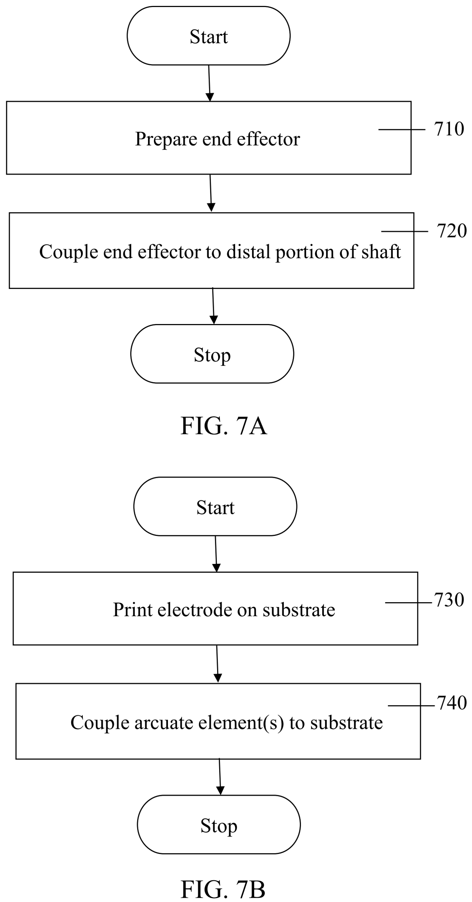

Reference is now made to FIG. 7A, which is a simplified flowchart of a method of manufacturing a catheter assembly having hotspot protection, according to examples of the presently disclosed subject matter. The catheter assembly includes at least one electrode. The electrode is coupled to at least one arcuate element, in order to reduce the intensity of the electric field at the respective location and prevent arcing.

In 710 the end effector is prepared. According to one aspect, the end effector is prepared as described with respect to FIG. 7B.

In 720 the end effector is coupled to the distal portion of a shaft that is configured to be guided to the ablation site through a delivery sheath.

Reference is now made to FIG. 7B, which is a simplified flowchart of a method of manufacturing an end effector, according to examples of the presently disclosed subject matter.

In 730, an electrode configured to deliver energy to an ablation site is printed on a substrate. The electrode has a bottom surface coupled to the substrate and an upper surface.

In 740, the bottom surface of at least one arcuate element is coupled to the upper surface of the electrode. The arcuate element(s) extend over respective locations so as to reduce an intensity of the electrical field at the respective location to below the arcing level during operation of the electrode in blood.

The term "upper surface of the electrode", according to some aspects, includes corners and edges of the electrode which are exposed and available for coupling to the arcuate element.

The coupled end effector and shaft provide a catheter assembly that has at least one protected location at which an arcuate element prevents the occurrence of hotspots during the operation of the electrode.

In one example, an arcuate element is coupled to the electrode by printing the arcuate element on the electrode.

Potential hotspot locations for an electrode without arcuate elements include but are not limited:
a) A location surrounding the junction between the substrate, electrode, and the blood;
b) A location surrounding the corner of electrode in the blood;
c) A location above the junction of the corner of the pad, the electrode, and the substrate.

According to an aspect of the disclosure at least one arcuate element extends over the location of a connection between the electrode and a power feed to the electrode.

According to an aspect of the disclosure at least one arcuate element extends over an edge of the top surface of the electrode.

According to an aspect of the disclosure at least one arcuate element extends over an exposed connection between the bottom surface of the electrode and the substrate.

According to an aspect of the disclosure, the arcuate elements cover less than 10% of the top surface of the electrode. In other aspects of the disclosure, the total area covered by the arcuate element(s) is less than 13% of the top surface of the electrode. In yet other aspects of the disclosure, the total area covered by the arcuate element(s) is less than 15% of the top surface of the electrode.

In one aspect of the disclosure, the electrode and the arcuate element are composed of the same material, thus having the same conductivity. The thickness of the arcuate element is within 1.8-5.2 times the thickness of the electrode. In one example, the electrode and the arcuate element have the same conductivity, which is between 10³-10⁶ S/m.

In another aspect of the disclosure, the electrode and arcuate element are composed of different materials and the conductivity of the arcuate element is less than the conductivity of the electrode. The thickness of the arcuate element is within 0.8-3.2 times the thickness of the electrode. In one example, the conductivity of the electrode is between 10³-10⁶ S/m and the conductivity of the arcuate element is within 0.5-5 S/m.

### Exemplary End Effectors

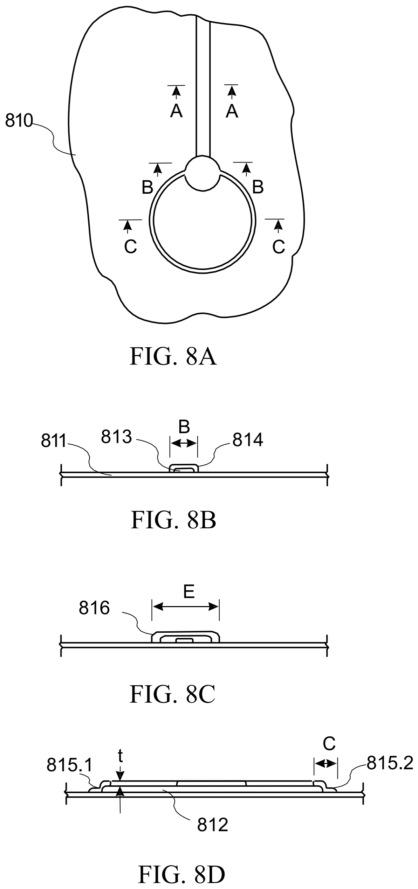

Reference is now made to FIGS. 8A-8D, which are simplified illustrations of an end effector, according to examples of the presently disclosed subject matter. For simplicity the electrode is presented having a round shape and printed on the wall of a balloon-shaped substrate. In alternate aspects, the electrode and substrate may be of other shapes and dimensions (e.g., the triangular electrodes of FIG. 5).

FIG. 8A is a top view of an electrode printed on the wall of a balloon-shaped substrate 810.

Figure 8B is cross-section view AA, showing a printed conductor 813 made of an electrically conductive material covered by an insulating material layer 814. Printed conductor 813 connects electrode 811 to the high input voltage supplied by an electric wire routed inside the balloon's shaft.

Figure 8C is cross-section view BB, showing an extended connection area covered by layer 16, with a diameter E between the conductor and the electrode. This area is required to reduce the high electric field generated at their connection point. The range of diameter E is 0.5 to 2 mm.

Figure 8D is cross-section view CC, showing electrode 812 coupled to two arcuate elements 815.1 and 815.2. The width of the arcuate elements is C.

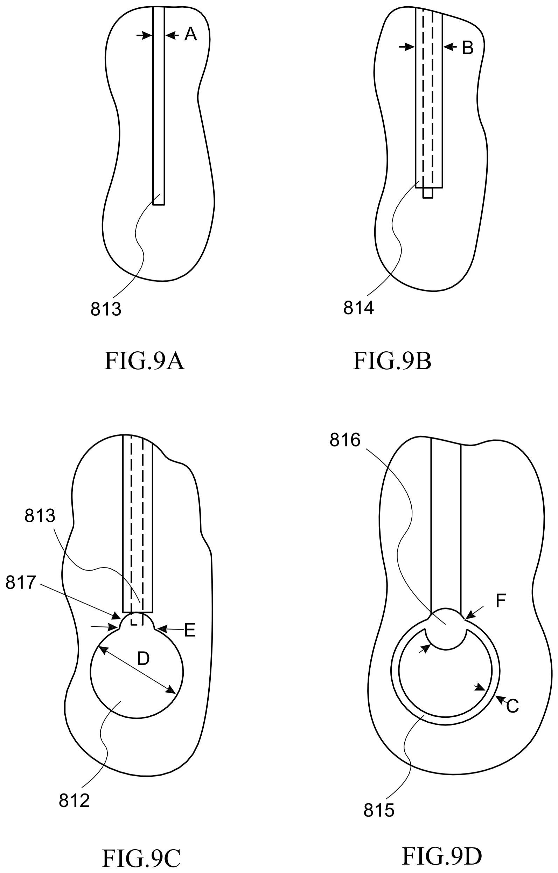

Reference is now made to FIGS. 9A-9D, which are simplified illustrations demonstrating a process for manufacturing the end effector of FIGS. 8A-8D, according to examples of the presently disclosed subject matter. The printing may be performed, for example, by spraying or pad printing.

Figure 9A shows a printed conductor 813 made from an electrically conductive material. In one example, printed conductor 813 has a typical thickness A of 10 microns (range 5 to 20 microns).

Figure 9B shows insulating material 814 printed over conductor 813 (to insulate it from the blood). The insulator does not cover the entire conductor 813, leaving part of it exposed. In one example, insulating material 814 has a typical thickness B of about 10 microns and a typical width of about 1 mm (range 0.5 to 2 mm).

Figure 9C shows an electrode printed on the exposed edge of the conductor 813. In one example, electrode 812 is made of a material with low to very low conductivity with a typical thickness of about 10 microns and a diameter D of about 10 mm. An extended connection area 817 of width E covers the connection between conductor 813 and electrode 812.

Figure 9D shows a protective arcuate element 815 printed around the edge and the extended connection area of electrode 812. In one example, the width C of arcuate element 815 is about 0.5-1.0 mm, and the diameter F of protection area 816 on the extended connection area is about 4 mm.

The types of material used for printing the arcuate element layer may be of either of the two approaches described with respect to FIGS. 6A and 6B. Namely arcuate element 815 may be made of the same material as electrode 812 or from a material with very low conductivity compared to the electrode material.

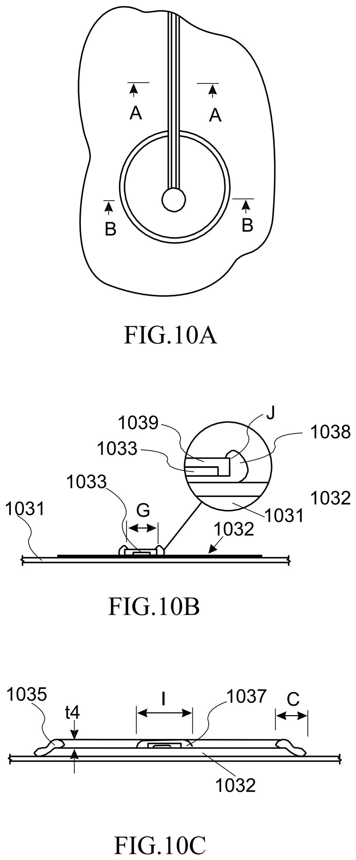

Reference is now made to FIGS. 10A-10C, which are simplified illustrations of an end effector, according to examples of the presently disclosed subject matter. FIG. 10A is a top view of an electrode printed on a balloon wall. FIG. 10B is cross-section view AA. FIG. 10C is cross-section view BB.

Electrode 1032 is printed on balloon wall 1031. Flexible printed circuit 1033 is made of an insulating base material 1039 (e.g., Kapton), and a metallic conductor 1033 (e.g., copper). Metallic conductor 1033 faces electrode 1032 and is adhered to it with conductive adhesive 1038. As shown in the inset of FIG. 10B, adhesive 1038 fills the gap between printed circuit 1033 and electrode 1032 and continues to spread to the sides, covering the sidewalls of printed circuit 1033 and extending beyond corner J.

Fig. 10C shows arcuate elements 1035 and 1037. Arcuate element 1035 extends along the entire edge of electrode 1032, covering both the top corner of electrode 1032 and the connection between electrode 1032 and printed circuit 1033. Arcuate element 1037 is located over the connection between conductor 1033 and electrode 1032. Arcuate element 1037 reduces the intensity of the electric field at the connection location, similarly to arcuate element 650 in Fig. 6A.

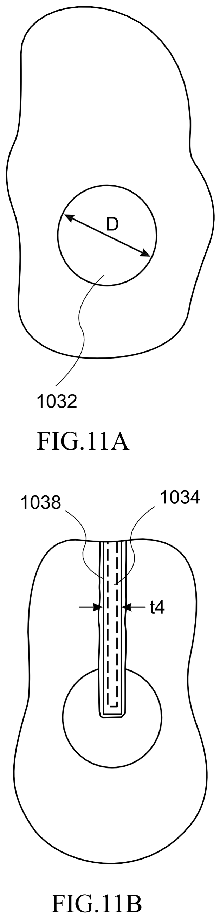

Reference is now made to FIGS. 11A-11D, which are simplified illustrations demonstrating the process for manufacturing the end effector of FIGS. 10A-10C, according to examples of the presently disclosed subject matter.

Figure 11A illustrates the printing of electrode 1032 itself with a diameter D, typically about 10 mm. The electrode material has low to very low electrical conductivity with a typical thickness of about 10 microns. FIG. 11B illustrates placing the flexible printed circuit 1033 and applying a layer of conductive adhesive 1038 to the bottom of the circuit. The adhesive layer 1038 is positioned on electrode 1032 so that its edge reaches approximately the center of the electrode and is pressed so that it spreads to the sides. Width t4 is typically about 1 mm.

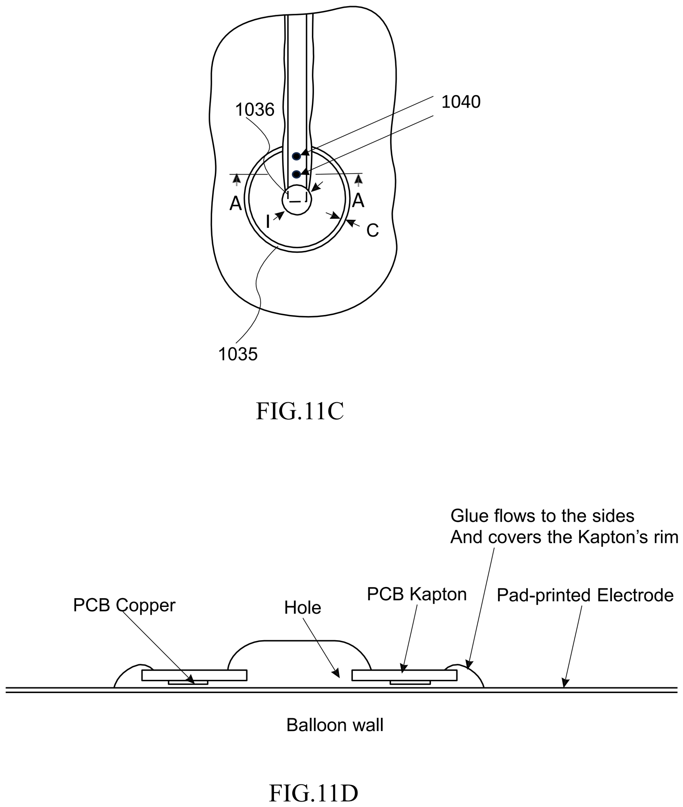

After proper drying of the adhesive, in FIG. 11C a protective layer is printed on the electrode edges 1035, with a typical width of about 0.5-1.0 mm. Arcuate element 1036 is dome-shaped and is printed with a diameter I of about 2-3 mm to protect against arcing on the printed circuit edge.

Optionally there are one or more hole(s) 1040 in printed circuit 1033. Hole(s) 1040 may be used to create arcuate element(s) on the edges of the electrode by injecting a conductive glue through the hole, as shown, for example, in Fig. 11D.

Fig. 11D is a cross-section view of AA of Fig. 11C, illustrating an example in which a metal-based adhesive (e.g. platinum or silver) is inserted into the gap between the printed circuit (PCB Copper) and the pad-printed electrode by injecting the adhesive through a hole in the printed circuit using a dispenser from the top side.

The types of material used for the arcuate elements may be of either of the two approaches described with respect to FIGS. 6A and 6B. Namely arcuate element 1035 may be made of the same material as electrode 1032 or from a material with very low conductivity compared to the electrode material.

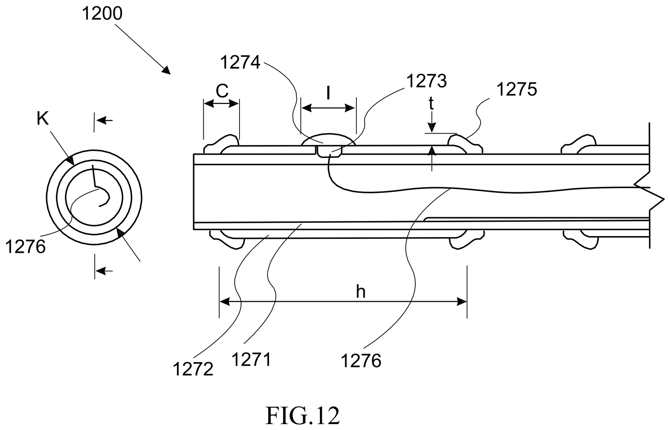

Reference is now made to FIG. 12, which is a simplified illustration of an edge effector, according to examples of the presently disclosed subject matter. Edge effector 1200 has a cylindrical form, which is an axisymmetric rotation of the flat end effectors illustrated in FIGS. 6A-6B. Substrate 1271 is a tube with a diameter between 1 and 3 mm. The tube has an electrode 1272 printed on it, made of a material with low to very low electrical conductivity, with a typical thickness of about 10 microns (range 5 to 50 microns). Thin layers enable maintaining the end effector's flexibility, especially for the small diameter catheters of about 1 mm required to be inserted into narrow blood vessels. The high voltage reaches electrode 1272 from metallic pad 1273, to which a thin electric wire is connected, routed inside the catheter toward the catheter handle (and from there through a connector to the generator). An arcuate element 1275 is applied around the electrode edges, with a thickness t of about 10-20 microns. Above the pad area, a dome-shaped arcuate element 1274 is applied with a diameter I slightly larger than the pad size.

The types of material used for the arcuate elements may be of either of the two approaches described with respect to FIGS. 6A and 6B. Namely arcuate elements 1275 and 1274 may be made of the same material as electrode 1032 or from a material with very low conductivity compared to the electrode material.

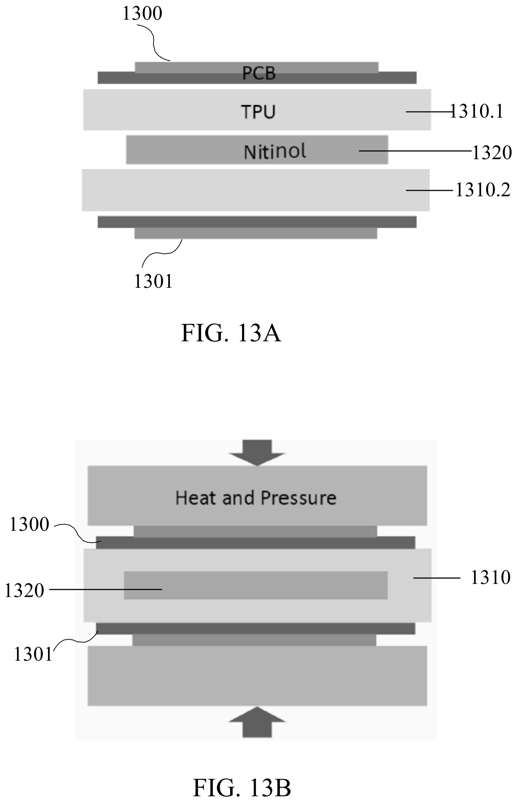

Reference is now made to FIGS. 13A-13C, which are simplified cutaway views of an embedded electrode illustrating the catheter's distal end lamination manufacturing process, according to an exemplary embodiment of the presently disclosed subject matter.

The catheter's distal end lamination process uses proud electrodes as "mold" shutoffs. During lamination, heat and pressure are applied to press the printed circuit board (PCB) into the thermoplastic polyurethane (TPU). The TPU flows up to the electrodes leaving the metal exposed.

FIG. 13A is a simplified illustration of layers stacked in preparation for the lamination process. The layered structure is:
i. Upper PCB with printed electrode 1300;
ii. Upper TPU layer 1310.1;
iii. Nitinol layer 1320;
iv. Lower TPU layer 1310.2; and
v. Lower PCB with printed electrode 1301.

In FIG. 13B, heat and pressure are applied to the stacked layers. As a result, the TPU layers fuse into a single TPU element 1310 which encloses Nitinol layer 1320.

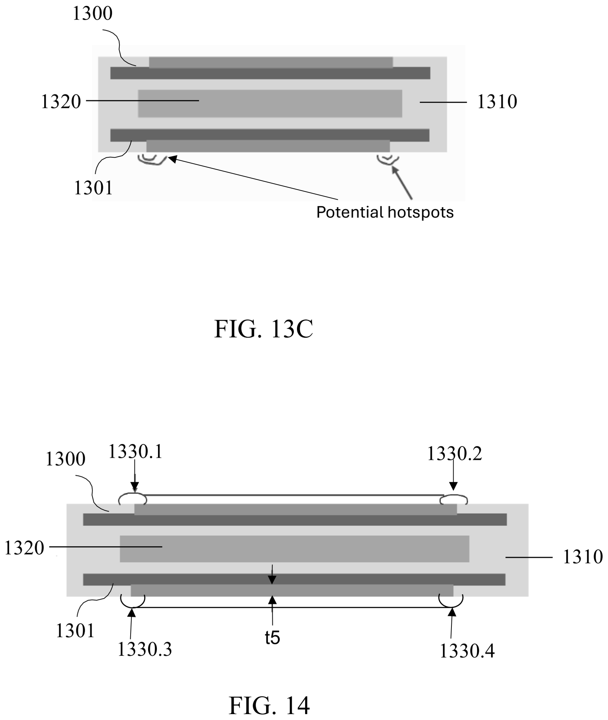

In FIG. 13C, heat and pressure continue to be applied. TPU element 1300 covers the sides of PCBs 1300 and 1301, leaving the conductive surfaces of the electrodes substantially exposed.

A problem may arise when the TPU does not meet the electrode edge, leaving the edges exposed. This creates potential hotspots at the interfaces between the embedded electrodes and the TPU.

Reference is now made to FIG. 14, which is a simplified cutaway view of an embedded electrode, according to an exemplary embodiment of the presently disclosed subject matter. Arcuate elements 1330.1-1330.4 are printed over respective interfaces between the electrodes and TPU 1310, such that the potential hotspot locations are fully covered.

In one aspect of the disclosure, the electrode and the arcuate element are composed of the same material, thus having the same conductivity. The thickness of the arcuate element is within 1.8-5.2 times the thickness of the electrode. In one example, the electrode and the arcuate element have the same conductivity, which is between 10³-10⁶ S/m.

In another aspect of the disclosure, the electrode and arcuate element are composed of different materials and the conductivity of the arcuate element is less than the conductivity of the electrode. The thickness of the arcuate element is within 0.8-3.2 times the thickness of the electrode. In one example, the conductivity of the electrode is between 10³-10⁶ S/m and the conductivity of the arcuate element is within 0.5-5 S/m.

In another example, the thickness of the electrode t5 is about 7-15µm and the thickness of the conductive printed arcuate elements is about 10-30µm.

### SUMMARY

Following is a non-exclusive list of some exemplary examples of the disclosure. The present disclosure also includes examples which include fewer than all the features in an example and examples using features from multiple examples, even if not listed below.

### Example 1:

A catheter assembly (400) configured for preventing an occurrence of at least one hotspot during operation of the catheter assembly (400), wherein a hotspot comprises a location at which a surrounding electrical field is above an arcing level in blood, includes:
a shaft (410) defining a longitudinal axis extending from a proximal portion to a distal portion of the shaft (410), the shaft (410) being configured to be guided into a lumen through a delivery sheath; and
an end effector (430) coupled to the distal portion of the shaft (410), the end effector (430) including:
an electrode (440) printed on a substrate (420), the electrode (440) having a bottom surface coupled to the substrate (420) and an upper surface, wherein the electrode (440) is configured to deliver energy to an ablation site; and
at least one arcuate element (450) having a bottom surface coupled to the upper surface of the electrode (440) and a curved upper surface, wherein the at least one arcuate element (450) extends over a respective location so as to reduce an intensity of the electrical field at the respective location to below the arcing level when the electrode (440) is operated in the blood, thereby preventing the occurrence of the at least one hotspot during operation of the electrode (440) in blood.

### Example 2:

The catheter assembly according to Example 1, further including a conductive pad configured to supply power to the electrode (440), wherein at least one of the arcuate elements (450) extends over a location of the conductive pad.

### Example 3:

The catheter assembly according to Example 1 or Example 2, wherein at least one of the arcuate elements (450) extends over an edge of the top surface of the electrode (440).

### Example 4:

The catheter assembly according to any one of Examples 1-3, wherein at least one of the arcuate elements (450) extends over an exposed connection between the bottom surface of the electrode (440) and the substrate (420).

### Example 5:

The catheter assembly according to any one of Examples 1-4, wherein the arcuate elements (450) cover less than 10% of the top surface of the electrode (440).

### Example 6:

The catheter assembly according to any one of Examples 1-4, wherein the arcuate elements (450) cover less than 13% of the top surface of the electrode (440).

### Example 7:

The catheter assembly according to any one of Examples 1-4, wherein the arcuate elements (450) cover less than 15% of the top surface of the electrode (440).

### Example 8:

The catheter assembly according to any one of Examples 1-7, wherein the electrode (440) and the at least one arcuate element are composed of the same material, and a thickness of the at least one arcuate element is within 1.8-5.2 times a thickness of the electrode (440).

### Example 9:

The catheter assembly according to any one of Examples 1-8, wherein the electrode (440) and the at least one arcuate element are composed of the same material, the material having a conductivity between 10³-10⁶ S/m.

### Example 10:

The catheter assembly according to any one of Examples 1-7, wherein a conductivity of the arcuate element is less than the conductivity of the electrode (440), and a thickness of the at least one arcuate element is within 0.8-3.2 times a thickness of the electrode (440).

### Example 11:

The catheter assembly according to any one of Examples 1-7 and 10, wherein the conductivity of the electrode (440) is between 10³-10⁶ S/m and the conductivity of the arcuate element is within 0.5-5 S/m.

### Example 12:

The catheter assembly according to any one of Examples 1-11, wherein the at least one arcuate element is printed on the upper surface of the electrode (440).

### Example 13:

The catheter assembly according to any one of Examples 1-12, wherein the substrate (420) is balloon-shaped.

### Example 14:

The catheter assembly according to any one of Examples 1-12, wherein the substrate (420) is cylindrical-shaped.

### Example 15:

A method of manufacturing a catheter assembly (400) having hotspot protection, wherein a hotspot comprises a location at which a surrounding electrical field is above an arcing level in blood, includes:
preparing an end effector (430) by:
printing an electrode (440) on a substrate (420), the electrode (440) having a bottom surface coupled to the substrate (420) and an upper surface, wherein the electrode (440) is configured to deliver energy to an ablation site; and
coupling at least one arcuate element to the electrode (440), the at least one arcuate element having a bottom surface coupled to the upper surface of the electrode (440) and a curved upper surface, wherein the at least one arcuate element extends over a respective location so as to reduce an intensity of the electrical field at the respective location to below the arcing level during operation of the electrode (440) in blood; and
coupling the end effector (430) to a distal portion of a shaft (410), the shaft (410) defining a longitudinal axis extending from a proximal portion to the distal portion of the shaft (410), the shaft (410) being configured to be guided to the ablation site through a delivery sheath,
thereby providing a catheter assembly (400) having at least one protected location for prevention of hotspot occurrence during the operation of the electrode (440).

### Example 16:

The method according to Example 15, wherein coupling at least one arcuate element to the electrode (440) includes printing the arcuate element on the electrode (440).

### Example 17:

The method according to Example 15 or Example 16, wherein at least one of the arcuate elements (450) extends over a location of a connection between the electrode (440) and a power feed to the electrode (440).

### Example 18:

The method according to any one of Examples 15-17, wherein at least one of the arcuate elements (450) extends over an edge of the top surface of the electrode (440).

### Example 19:

The method according to any one of Examples 15-18, wherein at least one of the arcuate elements (450) extends over an exposed connection between the bottom surface of the electrode (440) and the substrate (420).

### Example 20:

The method according to any one of Examples 15-19, wherein the arcuate elements (450) cover less than 15% of the top surface of the electrode (440).

### Example 21:

The method according to any one of Examples 15-20, wherein the electrode (440) and the at least one arcuate element are composed of the same material, and a thickness of the at least one arcuate element is within 1.8-5.2 times a thickness of the electrode (440).

### Example 22:

The method according to any one of Examples 15-20, wherein the conductivity of the electrode (440) is between 10³-10⁶ S/m, the conductivity of the arcuate element is within 0.5-5 S/m, and a thickness of the at least one arcuate element is within 0.8-3.2 times a thickness of the electrode (440).

Those skilled in the art to which the present disclosure pertains, can appreciate that while the present disclosure has been described in terms of preferred examples, the concept upon which this disclosure is based may readily be utilized as a basis for the designing of other structures, systems and processes for carrying out the several purposes of the present disclosure.

Also, it is to be understood that the phraseology and terminology employed herein are for the purpose of description and should not be regarded as limiting. It should be noted that the words "comprising", "including" and "having" as used throughout the appended claims are to be interpreted to mean "including but not limited to". The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases, and disjunctively present in other cases. The term "each" may not be exclusively understood as referring to each and every, and when technically relevant may also refer to "at least some".

All patents and patent applications mentioned in this specification are herein incorporated in their entirety by reference into the specification, to the same extent as if each individual patent or patent application was specifically and individually indicated to be incorporated herein by reference. In addition, citation or identification of any reference in this application shall not be construed as an admission that such reference is available as prior art to the present disclosure.

It is important, therefore, that the scope of the present disclosure is not construed as being limited by the illustrative examples set forth herein. Other variations are possible within the scope of the present disclosure as defined in the appended claims. Other combinations and sub-combinations of features, functions, elements and/or properties may be claimed through amendment of the present claims or presentation of new claims in this or a related application. Such amended or new claims, whether they are directed to different combinations or directed to the same combinations, whether different, broader, narrower or equal in scope to the original claims, are also regarded as included within the subject matter of the present description.

## Claims

1. A catheter assembly, said catheter assembly (400) being configured for preventing an occurrence of at least one hotspot during operation of said catheter assembly (400), wherein a hotspot comprises a location at which a surrounding electrical field is above an arcing level in blood, said catheter assembly (400) comprising:
a shaft (410) defining a longitudinal axis extending from a proximal portion to a distal portion of the shaft (410), the shaft (410) being configured to be guided into a lumen through a delivery sheath; and
an end effector (430) coupled to the distal portion of the shaft (410), the end effector (430) comprising:
an electrode (440) printed on a substrate (420), said electrode (440) having a bottom surface coupled to said substrate (420) and an upper surface, wherein said electrode (440) is configured to deliver energy to an ablation site; and
at least one arcuate element (450.1) having a bottom surface coupled to said upper surface of said electrode (440) and a curved upper surface, wherein said at least one arcuate element (450.1) extends over a respective location so as to reduce an intensity of said electrical field at said respective location to below said arcing level when said electrode (440) is operated in blood,
thereby preventing said occurrence of said at least one hotspot during operation of said electrode (440) in blood.

2. The catheter assembly of claim 1, further comprising a conductive pad configured to supply power to said electrode (440), wherein at least one of said arcuate elements (450.1-450.3) extends over a location of said conductive pad.

3. The catheter assembly of claim 1 or claim 2, wherein at least one of said arcuate elements (450.1-450.3) extends over an edge of said upper surface of said electrode (440).

4. The catheter assembly of any one of claims 1-3, wherein at least one of said arcuate elements (450.1-450.3) extends over an exposed connection between said bottom surface of said electrode (440) and said substrate.

5. The catheter assembly of any one of claims 1-4, wherein said arcuate elements (450.1-450.3) cover less than 15% of said upper surface of said electrode (440).

6. The catheter assembly of any one of claims 1-5, wherein said electrode (440) and said at least one arcuate element (450.1) comprise a same material, and a thickness of said at least one arcuate element (450.1) is within 1.8 to 5.2 times a thickness of said electrode (440).

7. The catheter assembly of any one of claims 1-6, wherein said electrode (440) and said at least one arcuate element (450.1) comprise a same material having a conductivity between 10³ to 10⁶ S/m.

8. The catheter assembly of any one of claims 1-5, wherein a conductivity of said arcuate element (450.1) is less than a conductivity of said electrode (440), and a thickness of said at least one arcuate element (450.1) is within 0.8 to 3.2 times a thickness of said electrode (440).

9. The catheter assembly of any one of claims 1-5 or of claim 8, wherein a conductivity of said electrode (440) is between 10³ to 10⁶ S/m and a conductivity of said arcuate element (450.1) is within 0.5 to 5 S/m.

10. The catheter assembly of any one of claims 1-9, wherein said at least one arcuate element (450.1) is printed on said upper surface of said electrode (440).

11. A method of manufacturing a catheter assembly having hotspot protection, wherein a hotspot comprises a location at which a surrounding electrical field is above an arcing level in blood, said method comprising:
preparing an end effector (430) by:
printing an electrode (440) on a substrate, said electrode (440) having a bottom surface coupled to said substrate and an upper surface, wherein said electrode (440) is configured to deliver energy to an ablation site; and
coupling at least one arcuate element (450.1) to said electrode (440), said at least one arcuate element (450.1) having a bottom surface coupled to said upper surface of said electrode (440) and a curved upper surface, wherein said at least one arcuate element (450.1) extends over a respective location so as to reduce an intensity of said electrical field at said respective location to below said arcing level during operation of said electrode (440) in blood; and
coupling said end effector (430) to a distal portion of a shaft (410), said shaft (410) defining a longitudinal axis extending from a proximal portion to said distal portion of the shaft (410), the shaft (410) being configured to be guided to said ablation site through a delivery sheath,
thereby providing a catheter assembly having at least one protected location for prevention of hotspot occurrence during said operation of said electrode (440).

12. The method of claim 11, wherein at least one of said arcuate elements (450.1-450.3) extends over at least one of: a location of a connection between said electrode (440) and a power feed to said electrode (440), an edge of said upper surface of said electrode (440) and an exposed connection between said bottom surface of said electrode (440) and said substrate.

13. The method of claim 11 or claim 12, wherein said arcuate elements (450.1-450.3) cover less than 15% of said upper surface of said electrode (440).

14. The method of any one of claims 11-13, wherein said electrode (440) and said at least one arcuate element (450.1) comprise a same material, and a thickness of said at least one arcuate element (450.1) is within 1.8 to 5.2 times a thickness of said electrode (440).

15. The method of any one of claims 11-13, wherein a conductivity of said electrode (440) is between 10³ to 10⁶ S/m, a conductivity of said arcuate element (450.1) is within 0.5 to 5 S/m, and a thickness of said at least one arcuate element is within 0.8 to 3.2 times a thickness of said electrode (440).
